(19) Europäisches Patentamt
European Patent Office
Office européen des brevets

(11) Publication number: **0 149 140**
**A2**

# (12) EUROPEAN PATENT APPLICATION

(21) Application number: 84115269.7

(22) Date of filing: 12.12.84

(51) Int. Cl.⁴: C 07 D 233/50
A 61 K 31/415

(30) Priority: 20.12.83 GB 8333835

(43) Date of publication of application: 24.07.85 Bulletin 85/30

(84) Designated Contracting States: BE CH DE FR GB IT LI NL SE

(71) Applicant: BEECHAM GROUP PLC
Beecham House Great West Road
Brentford Middlesex TW8 9BD(GB)

(72) Inventor: Newsome, Peter Martin
20A York Road
Cheam Surrey(GB)

(72) Inventor: Moss, Stephen Frederick
40 Blakehall Road
Carshalton Surrey(GB)

(74) Representative: Hardman, Carol Pauline et al,
Beecham Pharmaceuticals Great Burgh Yew Tree
Bottom Road
Epsom Surrey KT18 5XQ(GB)

(54) **Novel compounds.**

(57) Compounds of formula (I)

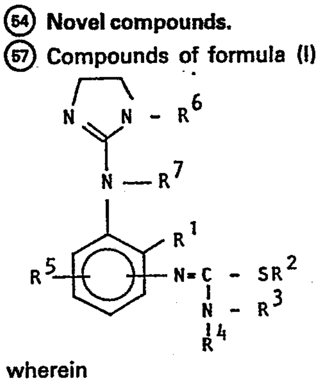

(I)

wherein
$R^1$ is halogen, $C_{1-4}$ alkyl or $C_{1-4}$ alkoxy;
$R^2$ is hydrogen, $C_{1-4}$ alkyl or optionally substituted aryl;
$R^3$ and $R^4$ are the same or different and each is hydrogen, $C_{1-6}$ alkyl, optionally substituted aryl or optionally substituted aralkyl;
$R^5$ is hydrogen, halogen, $C_{1-4}$ alkyl or $C_{1-4}$ alkoxy and
$R^6$ and $R^7$ are the same or different and each is hydrogen, $C_{1-4}$ alkyl or acyl
and acid addition salt thereof, protect animals from death due to scours.

EP 0 149 140 A2

## NOVEL COMPOUNDS

The present invention relates to a class of novel 2-phenylaminoimidazolines and to their use in human and veterinary medicine.

European Patent Application No. 0 043 659 discloses various 2-phenylaminoimidazolines bearing a nitrogen containing substituent on the phenyl ring which are useful in the treatment of diarrhoea and scours.

It has now surprisingly been found that a particular class of compounds falling within the generic disclosure of EP 0 043 659, but not specifically disclosed therein, have particularly advantageous properties.

Accordingly the present invention provides a compound of formula (I):

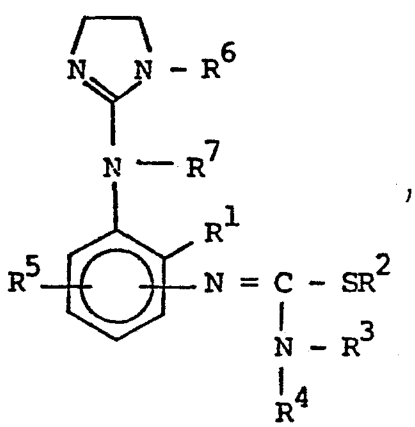

(I)

or acid addition salts thereof

wherein

$R^1$ is halogen, $C_{1-4}$ alkyl or $C_{1-4}$ alkoxy;

$R^2$ is hydrogen, $C_{1-4}$ alkyl or optionally substituted aryl;

$R^3$ and $R^4$ are the same or different and each is hydrogen, $C_{1-6}$ alkyl, optionally substituted aryl or optionally substituted aralkyl;

$R^5$ is hydrogen, halogen, $C_{1-4}$ alkyl or $C_{1-4}$ alkoxy and

$R^6$ and $R^7$ are the same or different and each is hydrogen, $C_{1-4}$ alkyl or acyl.

Suitable aryl groups include phenyl and naphthyl groups. Suitable aralkyl groups include phenyl $(C_{1-4})$ alkyl groups, especially benzyl groups.

Suitable substituents for aryl and aralkyl groups include halogen, alkyl, alkoxy, alkylthio, amino, mono- and di-alkylamino, mono- and di-acylamino, nitro, cyano, hydroxy, hydroxyalkyl, alkoxyalkyl, mercapto, mercaptoalkyl, alkylthioalkyl, amidino, guanidino, sulphonyl, sulphonamido or carboxy or an ester or amide of carboxy.

Suitable acid addition salts include the pharmaceutically or veterinarily acceptable salts of acids such as hydrochloric, hydrobromic, hydroiodic, nitric, sulphuric, citric, tartaric and pamoic acids.

As used herein the terms 'alkyl' and 'alkoxy' refer to straight chain, branched chain or cyclic groups; straight chain and branched chain groups preferably having from 1 to 4 carbon atoms, cyclic groups preferably having 5 or 6 carbon atoms.

As used herein the term 'acyl' refers to the residues of carboxylic acids, particularly alkanoic acids having from 1 to 4 carbon atoms and optionally substituted benzoic acids.

When $R^7$ is hydrogen the compounds of formula (I) may exist either as the phenylaminoimidazoline form or the tautomeric phenyliminoimidazolidine form and when one of $R^3$ and $R^4$ is hydrogen the isothiourea substituent may exist in the alternative isothiourea form or, when $R^2$ is hydrogen, it may exist as the thiourea. All tautomers of the compounds of formula (I) are encompassed by the invention.

Suitably, the invention also includes a solvate of a compound of formula (I), preferably a hydrate.

Preferably $R^1$ is halogen, especially chlorine, or alkyl, especially methyl.

Preferably $R^5$ is in the ortho or meta position with respect to the imidazolinamino moiety.

Preferably $R^5$ is halogen, especially chlorine, or alkyl, especially methyl.

Preferably the group $-N{=}C{-}SR^2$ is in the para
|
$NR^3R^4$

position with respect to the imidazolinamino group.

A particularly preferred class of compounds within formula (I) are those having formula (IA):

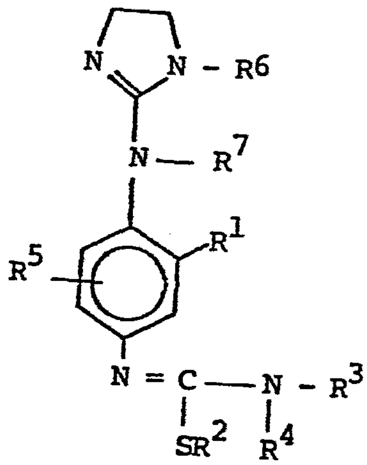

(IA)

or acid addition salts thereof,

wherein

$R^1$, $R^2$, $R^3$, $R^4$, $R^5$, $R^6$ and $R^7$ are as defined in relation to compounds of formula (I).

Most preferably $R^1$ and $R^5$ are chlorine or methyl and $R^2$ is hydrogen or methyl, $R^3$ is hydrogen and $R^4$ is methyl, phenyl, benzyl or substituted phenyl or benzyl.

The present invention also provides a process for producing a compound of formula (I) as herein before defined, which process comprises

(a) reacting a compound of formula (II):

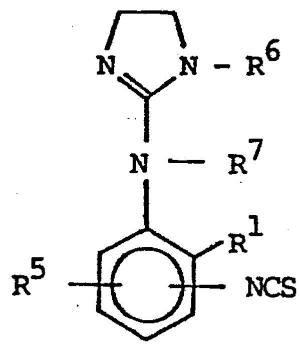

(II)

wherein $R^1$,$R^5$,$R^6$ and $R^7$ are as defined in relation to compounds of formula (I),

with an amine of formula (III):

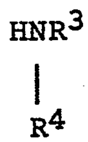

(III)

wherein $R^3$ and $R^4$ are as defined in relation to compounds of formula (I); or

(b) for compounds of formula (I) wherein $R^4$ is hydrogen, by reacting a compound of formula (IV):

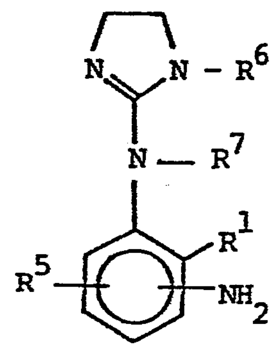

(IV)

wherein $R^1$, $R^5$, $R^6$ and $R^7$ are as defined in relation to compounds of formula (I),

with a compound of formula (V):

$SCN-R^3$ (V)

wherein $R^3$ is as defined in relation to compounds of formula (I);

to produce a thiourea of formula (I) and optionally thereafter converting the thiourea into an isothiourea of formula (I).

The reaction of a compound of formula (II) with a compound of formula (III) or of a compound of formula (IV) with a compound of formula (V) is preferably conducted in a suitable solvent such as a lower alkanol, optionally in the presence of water, at ambient or raised temperature.

The conversion of a thiourea into an isothiourea is suitable effected by reacting the thiourea with the appropriate alkyl or aryl halide, preferably the alkyl or aryl iodide, in a suitable solvent such as a lower alkanol at ambient or raised temperature.

The compounds of formula (I) may be separated from the reaction mixture and purified by conventional methods.

With the exception of paraisothiocyanato clonidine the compounds of formula (II) are novel and, being useful as intermediates, form a further aspect of the invention.

Accordingly the present invention provides a compound of formula (II):

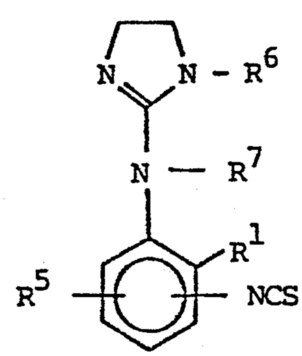

or acid addition salts thereof,

wherein $R^1$, $R^5$, $R^6$ and $R^7$ are as defined in relation to compounds of fomula (I),

provided that when $R^6$ and $R^7$ are hydrogen
and $R^1$ and $R^5$ are chloro
and $R^5$ is in the ortho position with respect to the imidazolinamino group
then the isocyanato group is in the meta position with respect to the imidazolinamino group.

Preferred compounds of formula (II) are those corresponding with preferred compounds of formula (I).

The compounds of formula (II) may be prepared by reacting a compound of (IV) :

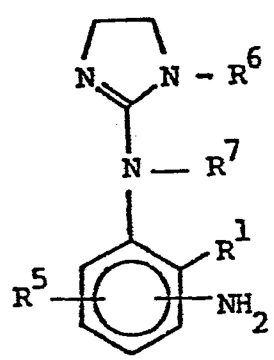

(IV)

or an acid addition salt thereof, wherein $R^1$, $R^5$, $R^6$ and $R^7$ are as defined in relation to formula (I), with a compound of formula (IIA):

$$Q_2C = S \qquad \text{(IIA)}$$

wherein Q is a leaving group for example halogen. Preferably Q is chlorine.

The reaction between compounds of formula (IV) and (IIA) may be carried out in any suitable solvent system, including mixed solvent systems of mutually immiscible solvents, at temperatures between 0°C and 50°C. Suitably an aqueous solution of an acid addition

salt, for example the dihydrochloride, of a compound of formula (IV) is admixed with a solution of the compound of formula (IIA) in a water immiscible solvent, for example chloroform, at room temperature.

The compounds of formula (II) may be separated from the reaction mixture and, if necessary, purified by conventional methods.

The compounds of formula (IV) and (IIA) are known compounds and may be prepared by methods conventional in the art.

Compounds of formula (I) reduce the mortality associated with enteropathogenic E.coli infections and are therefore useful in the treatment of diarrhoea and scours of humans and animals.

Accordingly the present invention also provides a compound of formula (I) for use in the treatment of the human or animal body, especially for the treatment of enteropathogenic diarrhoea and scours.

The present invention also provides a pharmaceutical or veterinary formulation comprising a compound of formula (I) or an acid addition salt thereof as hereinbefore defined (hereinafter referred to as the 'drug') and a pharmaceutically or veterinarily acceptable carrier therefor.

Pharmaceutical and veterinary compositions of the 'drug' will, of course, be adapted for administration to the humans or animals to be treated. Thus, for example, the composition may be a shaped composition, such as a bolus, tablet or capsule. In such cases the pharmaceutically or veterinarily acceptable carrier

will be chosen from the usual range of lubricants, dispersants, binders, fillers and the like. When these shaped compositions are for administration to cattle and pigs often they may for instance weigh at least 1 g, on occasions at least 2 g.

For administration to humans, especially children, the drug may suitably be presented as a syrup including suitable colouring and/or flavouring agents. Such syrups are conveniently presented in unit or multi-dose containers.

For veterinary use the composition may also be a dispersion or a solution of the drug in a suitable vehicle for use with an oral doser (this is a well known item of farm equipment, basically comprising a liquid reservoir, a mouthpiece adapted for insertion into animals mouths, and a pump mechanism whereby unit doses can be ejected from the reservoir through the mouthpiece). Conveniently the drug may be administered from an oral doser as an aqueous solution. Alternatively, the vehicle will be an oil or water based cream to ensure homogeneity of the unit doses administered.

The invention, therefore, also provides an oral doser containing a multi-dose of the drug in a veterinarily acceptable vehicle.

The drugs of the invention may also be added to the animal feed or drinking water. It will be convenient to formulate these animal feed and drinking water compositions with a multi-dose of the drug so that the animal takes in an appropriate quantity of the drug along with its diet. It will also be convenient to present the composition of the invention as a premix for addition to the feed or drinking water.

With young animals, a particularly useful technique is to blend the drug with the milk provided for them.

The compositions of the invention may also be formulated for injection. In such cases the drug chosen is suitably dissolved in water for injection. Alternatively the drug may be administered in a solution used for parenteral fluid replacement therapy.

Treatment of diarrhoea and scours using the drug may be supplemented by oral rehydration therapy such as those described in U.K. Patent No. 1,581,826 and German Offenlegungsschrift No. 28 54 281, UK Patent Application No. 2 012 163A, US Patent No. 3 898 328, Nalin, D.R. and Cash, R.A., Bull. World Health Org., 43, 361 (1970), French Patent No. 2 467 599, UK Patent No. 1 465 308 and as described in 'Secretory Diarrhoea', Ed M. Field, J.S. Fordtran and S.G. Schultz, American Physiological Society, Maryland, 1980 pp 179-185 and Lancet, (1975) pp 79 and 80. Conveniently the drug may be administered with the oral rehydration formulation.

Accordingly the present invention provides, in a particular aspect, a formulation for treating diarrhoea which comprises an effective non-toxic amount of a compound of formula (I) or an acid addition salt thereof, as hereinbefore defined and an oral rehydration composition comprising a pharmacologically acceptable aqueous solution containing at least 0.5% w/v of an actively absorbed monosaccharide, at least 25 mM sodium ions and having an osmolarity less than 500 m Osmolar.

Preferably the oral rehydration composition further comprises electrolyte and actively-absorbed amino

acid. Suitable actively-absorbed monosaccharides include glucose and suitable actively-absorbed amino acids include glycine.

The drug may be presented as a formulation containing one or more components of the oral rehydration composition for admixture with the remaining components.

Alternatively the drug may be provided separately and administered simultaneously or sequentially with the oral rehydration formulation.

Often it will be appropriate to include in the compositions a further medicine such as an antibacterial agent for example an antibiotic such as amoxycillin or neomycin or a sulphonamide such as sulfadoxin, an agent to alter intestinal motility such as loperamide or a material such as pectin.

The amount of drug administered must, of course, be sufficient to bring about the desired effect and will also depend on the body weight of the recipient and the chosen route of administration. It is believed that a suitable dose of the drug for use in the present treatment, would be in the range 0.001 to 10mg/kg, which dose may be repeated as and when required. Particularly suitable ranges include 0.01 to 2 mg/kg and especially 0.05 to 2 mg/kg. Nevertheless it will be appreciated that the dose used in practice will depend on the species to which the dose is administered and on the route of administration. Useful dosage units based on such dosage would contain from 0.001 mg to 100 mg of the drug, more suitably 0.05 mg to 100 mg. Of course, it will be appreciated that many

preferred compositions of the invention are in multi-dose form as, for the therapy of animals, it is often most desirable to be able rapidly to treat a number of animals. Such multi-dose compositions will contain, by way of example, at least 1 mg of the drug. Depending on the exact nature of the said multi-dose composition, often it will contain at least 25 mg of the drug, and on occasions as much as 2.5 g. Doses may be administered once or several times daily.

Within the above indicated dosage ranges, no adverse toxicological effects have been observed with the compounds of the invention.

The present invention further provides a method for treating diarrhoea or scours which comprises administering a compound of formula (I), or an acid addition salt thereof, as hereinbefore defined, to a human or non-human animal in need thereof.

In a particular embodiment the invention provides a method for treating diarrhoea or scours which comprises administering a pharmaceutical or veterinary composition as hereinbefore defined, particularly an oral rehydration composition.

The invention will now be illustrated by the following Examples, Descriptions, Biological Data and Formulations. As used in the Formulations the term 'drug' refers to the compound of Example I or an acid addition salt thereof; in the case of such salts the quantity of drug is calculated on the basis of the equivalent weight of free base unless otherwise stated.

## Examples 1 to 7

### Preparation of phenylimidazolidine thioureas

a) Phenylimidazolidine-N-methylthioureas were prepared by stirring the appropriate phenyl imidazolidine isothiocyanate hydrochloride (0.5 mol), and a 25% aqueous solution of the appropriate alkylamine (5 mol) in ethanol (1 l) for 24 h. The solvent was evaporated *in vacuo* to leave the product which was purified either by chromatography or trituration of the corresponding hydrochloride derivative in acetone.

b) Phenylimidazolidine-N-benzylthioureas were prepared by stirring equimolar amounts of the appropriate phenylimidazolidine hydrochloride with the benzylamine in ethanol for 24h. The solvent was evaporated to leave the product which was purified by trituration of the corresponding hydrochloride derivative in either acetone or acetonitrile.

### Example 1

### 2-[2,6-Dimethyl-4-(1-methyl-3-thioureido)phenylimino]-imidazolidine hydrochloride

2-(4-Isothiocyanato-2,6-dimethylphenylimino)-imidazolidine hydrochloride was treated with 25-30% aqueous methylamine as described. The title compound was obtained (mpt. 225°C) by trituration with acetone.

Analysis calculated for $C_{13}H_{20}ClN_5S$.

Theory: C, 49.76, H, 6.43; N, 22.31; S, 10.20%
Found : C, 49.76; H, 6.45; N, 21.98; S, 10.15%

Example 2

2-[2-Methyl-5-(1-methyl-3-thioureido)phenylimino]-imidazolidine hydrochloride

2-(5-Isothiocyanato-2-methylphenylimino)imidazolidine hydrochloride was treated with 25-30% aqueous methylamine as described. The title compound was purified by column chromatography on silica gel, using a methanol-dichloromethane eluant gradient, to yield white prisms (mpt. 181-6$^{o}$C (dec)).

$^{1}$H nmr (60 MHz) $\delta[(CD_3)_2SO]$

10.2 (br, 1H, $D_2O$-labile, $N^{+}H=$), 8.5 (br, 3H, $D_2O$-labile, 3NH), 7.50 (M, 3H, ArH), 3.60 (S, 4H, $(CH_2)_2$), 3.50 (br, NH, integration masked by $H_2O$), 2.95 (d, J = 4Hz, 3H, NH$\underline{CH_3}$), 2.20 ppm (S, 3H, $ArCH_3$).

Example 3

2-[2,6-Dimethyl-4-(1-[4-fluorobenzyl]-3-thioureido)-phenylimino]imidazolidine hydrochloride

2-(4-Isothiocyanato-2,6-dimethylphenylimino)-imidazolidine hydrochloride was treated with 4-fluorobenzylamine as described. The title compound (mpt. 213-18$^{o}$C) was obtained by trituration with acetone.

Analysis calculated for $C_{19}H_{23}ClFN_5S$.
Theory : C, 55.94; H, 5.68; N, 17.17; S, 7.86%.
Found : C, 55.33; H, 5.93; N, 16.89; S, 7.91%.

Example 4

2-[2,6-Dichloro-4-(1-methyl-3-thioureido)phenylimino]-imidazolidine hydrochloride

2-(2,6-Dichloro-4-isothiocyanatophenylimino)-imidazolidine hydrochloride was treated with 25-30% aqueous methylamine as described. The title compound (mpt. 210-13°C), was obtained by trituration with acetonitrile.

$^{1}H$ nmr (60MHz) $\delta[(CD_3)_2SO-D_2O]$
7.70 (s, 2H, ArH), 3.50 (S, 4H, $(CH_2)_2$), 2.90 ppm. (S, 3H, $NH\underline{CH_3}$).

Example 5

2-[2,6-Dichloro-4-(1-[4-fluorobenzyl]-3-thioureido)-phenylimino]imidazolidine hydrochloride

2-(2,6-Dichloro-4-isothiocyanatophenylimino)-imidazolidine hydrochloride was treated with 4-fluorobenzylamine as described. The title compound was obtained by trituration with acetonitrile.

Analysis calculated for $C_{17}H_{17}Cl_3FN_5S$
Theory : C, 45.51; H, 3.82; N, 15.60; S, 7.13%.
Found : C, 45.57; H, 3.71; N, 15.34; S, 6.81%.

Example 6

2-[4-(1-Benzyl-3-thioureido)-2,6-dichlorophenylimino]-imidazolidine hydrochloride

2-(4-Isothiocyanato-2,6-dichlorophenylimino)-imidazolidine hydrochloride was treated with

benzylamine as described. The title compound (mpt. 175°C) was obtained by trituration with acetonitrile.
Analysis calculated for $C_{17}H_{18}Cl_3N_5S$.
Theory : C, 47.40; H, 4.21; N, 16.25; S, 7.42%.
Found : C, 47.27; H, 4.18; N, 16.07; S, 7.45%.

Example 7

2-[2,6-Dichloro-4-(1,1-dimethyl-3-thioureido)-phenylimino]imidazolidine hydrochloride

2-(2,6-Dichloro-4-isothiocyanatophenylimino)-imidazolidine hydrochloride was treated with 25% aqueous dimethylamine as described. The title compound was purified by column chromatography on silica gel using a methanol-dichloromethane eluant gradient, to yield a pale yellow solid.

$^1H$ nmr (90 MHz) $\delta[(CD_3)_2SO - D_2O]$
7.30 (S, 2H, ArH), 3.30 (S, 4H, $(CH_2)_2$), 3.20 ppm (S, 6H, $N(CH_3)_2$).

Example 8

2-[4-(1-Benzyl-3-thioureido)-2,6-dimethylphenylimino]-imidazolidine hydrochloride

2-(4-Isothiocyanato-2,6-dimethylphenylimino)-imidazolidine hydrochloride was treated with benzylamine as described. The title compound was obtained by trituration with acetonitrile.

Example 9

2-[2,6-Dichloro-4-(1-phenyl-3-thioureido)-phenylimino]imidazolidine hydrochloride

2-(2,6-Dichloro-4-isothiocyanatophenylimino)-imidazolidine hydrochloride was treated with phenylamine as described. The title compound was obtained by trituration with acetonitrile.

Example 10

2-[2,6-Dichloro-4(1-cyclohexyl-3-thioureido) phenylimino]-imidazolidine hydrochloride

2-(2,6-Dichloro-4-isothiocyanatophenylimino)-imidazolidine hydrochloride was treated with cyclohexylamine as described. The title compound was obtained by trituration with acetonitrile.

Examples 11-15

Preparation of phenylimidazolidine isothioureas

The appropriate phenylimidazolidine thiourea (0.5 mol), iodomethane (0.6 mol) and methanol (1 l) were stirred at room temperature for 24h. The solvent was evaporated under vacuum to yield crude product which was either purified as the hydrohalide derivative by trituration with acetone, or as the free base by column chromatography.

Example 11

2-[2,6-Dimethyl-4-(1,2-dimethyl-3-isothiouronio)-phenylimino]imidazolidine iodide hydrochloride

2-[2,6-Dimethyl-4-(1-methyl-3-thioureido)phenylimino]-imidazolidine hydrochloride was treated with iodomethane as described. The title compound (mpt. 215°C(dec)) was obtained by trituration with acetone.
Analysis calculated for $C_{14}H_{23}ClIN_5S$
Theory : C, 36.89; H, 5.09; N, 15.37; S, 7.03%.
Found : C, 36.21; H, 4.97; N, 14.80; S, 6.99%.

Example 12

2-[5-(1,2-Dimethyl-3-isothiouronio)-2-methyl)-phenylimino]imidazolidine iodide

2-[2-Methyl-5-(1-methyl-3-thioureido)phenylimino]-imidazolidine hydrochloride was treated with iodomethane as described. The title compound was purified by column chromatography on silica gel, using a methanol-dichloromethane eluant gradient to yield a white solid, (mpt. 171-6°C(dec)).
Analysis calculated for $C_{13}H_{20}IN_5S$.
Theory : C, 38.52; H, 4.97; N, 17.28; S, 7.91%.
Found : C, 38.48; H, 4.97; N, 16.92; S, 7.76%.

Example 13

2-[2,6-Dichloro-4-(1,2-dimethyl-3-isothiouronio)-phenylimino]imidazolidine hemihydrochloride

2-[2,6-Dichloro-4-(1-methyl-3-thioureido)phenylimino]-imidazolidine hydrochloride was treated with iodomethane as described. The title compound (mpt.

200°C (dec)) was purified by column chromatography on alumina using chloroform eluant, to yield a cream solid.

Analysis calculated for $C_{12}H_{15}Cl_2N_5S$. 0.5 HCl.

Theory:C, 41.20; H, 4.32; N, 20.00; S, 9.15; Cl 25.33%.

Found :C, 41.48; H, 4.28; N, 18.69; S, 8.80; Cl 25.72%.

## Example 14

### 2-[2,6-Dichloro-4-(1,1,2-trimethyl-3-isothioureido)-phenylimino]imidazolidine

2-[2,6-Dichloro-4-(1,1-dimethyl-3-thioureido)-phenylimino]imidazolidine hydrochloride was treated with iodomethane as described. The title compound was purified by column chromatography on alumina using chloroform as eluant, to yield a cream solid.

Analysis calculated for $C_{13}H_{17}Cl_2N_5S$. 0.3 HCl.

Theory:C, 43.7; H, 4.89; N, 19.59; Cl, 27.82; S, 8.96%.

Found:C, 44.29; H, 4.84; N, 19.66; Cl, 22.82; S, 8.94%.

## Example 15

### 2-[2,6-Dichloro-4-(1-[4-fluorobenzyl]-2-methyl-3-isothioureido)phenylimino]imidazolidine hemihydrate

2-[2,6-Dichloro-4-(1-[4-fluorobenzyl]-3-thioureido)-phenylimino]imidazolidine hydrochloride was treated with iodomethane as described. The title compound was purified by column chromatography on alumina using a methanol-dichloromethane eluant gradient, to yield a cream solid.

Analysis calculated for $C_{18}H_{18}Cl_2FN_5S$. 0.5 $H_2O$.

Theory : C, 49.66; H, 4.40; N, 16.08; S, 7.35%.

Found : C, 49.64; H, 4.14; N, 15.73; S, 7.01%.

## Descriptions 1 to 3

## Preparation of phenylimidazolidine isothiocyanates

A solution of the appropriate aminophenylimidazolidine dihydrochloride (0.5 mol) in 4M hydrochloric acid (2l) was added over 2h to a vigourously stirred solution of thiophosgene (0.63 mol) in chloroform (2l). After 20 h stirring the two phases were separated. The aqueous phase was cautiously basified to pH10 with saturated sodium carbonate solution and then further extracted with chloroform. Combined chloroform extracts were washed with sodium bicarbonate solution, water and then dried (magnesium sulphate) and evaporated. A solution of the resulting concentrate in either acetonitrile or acetone, when acidified with ethanolic hydrogen chloride yielded the hydrochloride of the appropriate title compound.

### Description 1

### 2-(5-Isothiocyanato-2-methylphenylimino)imidazolidine hydrochloride.

Thiophosgene was treated with 2-(5-amino-2-methylphenylimino)imidazolidine dihydrochloride as described. The title compound was isolated as a white solid, (mpt. 150-3°C).

Description 2

2-(2,6-dichloro-4-isothiocyanatophenylimino) imidazolidine hydrochloride

Thiophosgene was treated with 2-(4-amino-2,6-dichlorophenylimino)imidazolidine dihydrochloride as described. The title compound was isolated as colourless prisms from acetonitrile.

$^1H$ nmr (60 MHz) $\delta H[(CD_3)_2SO]$
8.67 (broad s, 2H, $D_2O$-labile, NH), 7.77 (S,2H,ArH), 3.63 ppm (S,4H, $CH_2.CH_2$).

Description 3

2-(4-Isothiocyanato-2,6-dimethylphenylimino)-imidazolidine hydrochloride

Thiophosgene was treated with 2-(4-amino-2,6-dimethylphenylimino)imidazolidine dihydrochloride as described. The title compound was isolated as colourless prisms from acetonitrile.

Analysis calculated for $C_{12}H_{15}ClN_4S$

Theory : C, 51.16; H, 5.01; N, 19.88; S, 11.31%.
Found : C, 51.29; H, 5.29; N, 19.69; S, 11.46%.

BIOLOGICAL DATA

Protection of Neonatal Mice from Lethal Enteropathogenic E coli Infection

4 day old mice were dosed orally with 50µl of phosphate buffered saline containing 1 x $10^5$ organisms/ml of *E coli* B44 (09:K90:K99) an enteropathogenic strain

originally isolated from a scouring calf. The mice were then dosed b.i.d. with either placebo or drug as the hydrochloride salt for four days commencing 16 hours after infection. The animals were left with their mothers throughout the experiment and a daily record of deaths were made. The experiment was terminated 10 days after infection of the mice.
The mortality in the drug group was then compared with the mortality in the placebo group using the following formula:-

$$\% \text{ Reduction in mortality} = \frac{Mp-Md}{Mp} \times 100$$

where Mp = mortality in group receiving placebo
Md = mortality in group receiving drug

Statistical analysis was performed using 2 x 2 contingency tables (single tailed 'p'). Results are given in Tables 1 and 2. 'Protection %' is the same as '% Reduction in mortality' above.

Table 1

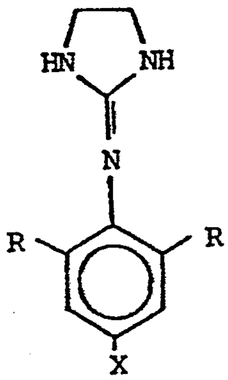

| R | X | dose* mg/kg | Protection (%) | No. Infected |
|---|---|---|---|---|
| Me | 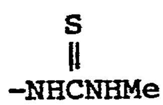 | 2 | 69 | 52(P<0.05) |
| | | 0.4 | 43 | 44 |
| Me | 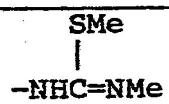 | 0.4 | 11 | 26 |
| Me | 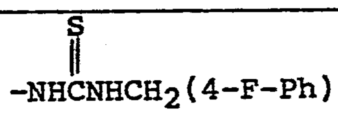 | 2 | 11 | 24 |
| Cl | 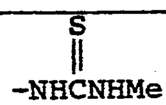 | 0.4 | 6 | 26 |
| | | 0.08 | 6 | 26 |
| Cl | 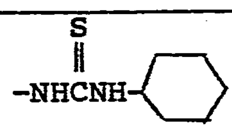 | 0.08 | 6 | 27 |
| Cl | 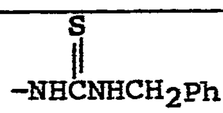 | 0.4 | 21 | 46 |
| Cl | 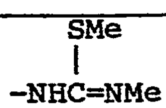 | 2 | 13 | 67 |
| | | 0.4 | 26 | 51(P<0.05) |
| | | 0.08 | 22 | 64 |
| Cl | 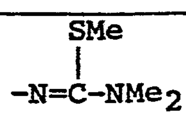 | 0.08 | 13 | 23 |
| Cl | 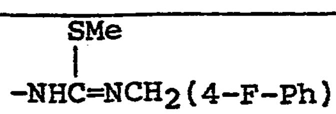 | 0.04 | 40 | 47(P<0.05) |
| | | 0.08 | 29 | 19 |

* Dose of hydrochloride salt

Table 2

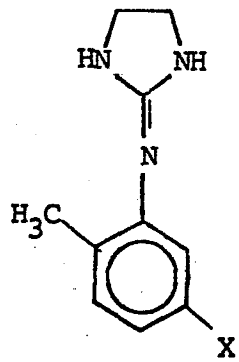

| X | Dose* mg/kg | Protection % | No. of Animals |
|---|---|---|---|
| 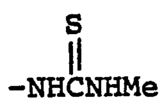 | 10 | 5 | 23 |
| 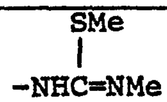 | 10 | 13 | 24 |

* Dose of hydrochloride salt

## Formulation of the Compounds for Veterinary Administration

### Formulation 1: Bolus

Boluses of the following composition may be prepared:-

| | |
|---|---|
| Drug | 16 mg |
| Microcrystalline cellulose | 500 mg |
| Corn starch | 250 mg |
| Magnesium stearate | 25 mg |
| Lactose, anhydrous | to 2500 mg |

The ingredients are passed through a 30 mesh stainless steel screen and blended in a suitable blender. The resultant compression mix is compressed directly on a tabletting machine to give tablets each containing 16 mg of the drug.

### Formulation 2:

### Oral Doser

1 kg of the following composition may be prepared:-

| | % by wt. |
|---|---|
| Drug | 0.02 |
| Aluminium stearate | 6.0 |
| Sunflower oil | to 100 |

The aluminium stearate is dispersed with stirring in a portion of the sunflower oil heated to 115°C. The dispersion is added to the rest of the sunflower oil

heated to 140$^{o}$C. The gel is stirred at 130$^{o}$C for 15 minutes and then allowed to cool without stirring to room temperature. The milled drug is dispersed in the cooled gel base and then passed through a colloid mill to produce a fine, homogenous dispersion. The dispersion is filled into plastic bottles fitted with a dosing pump.

Formulation 3

Injection

1 litre of the following composition may be prepared:-

| | % w/v |
|---|---|
| Drug | 0.1 |
| Sodium chloride | 0.9 |
| Water for injections | to 100 |

The drug and sodium chloride are dissolved in the water for injections and the solution is sterilised by membrane filtration and filled into glass ampoules.

Formulation 4

Soluble Powder

1 Kg of the following composition may be prepared:-

| | % by wt. |
|---|---|
| Drug | 0.1 |
| Lactose | to 100 |

The drug and lactose are sieved and mixed together in a suitable blender to give a homogenous powder. The powder is filled into jars. The powder is used at the rate 1 g per gallon of drinking water to medicate pigs.

Formulation 5

Oral Rehydration Formulation

1 kg of the following composition may be prepared by mixing together the ingredients in dry powder form:

| | |
|---|---|
| Glycine | 10.3 % |
| Dextrose (anhydrous) | 67.6 |
| Sodium Chloride | 14.3 |
| Potassium Dihydrogen Phosphate | 6.8 |
| Citric Acid | 0.8 |
| Tri-potassium Citrate | 0.2 |
| Drug | 0.05 |

60 g of the composition is then dissolved in 2 litres of water and fed to diarrhoeic calves.

Formulation 6

The following formulation may be prepared by the method set out below:-

| | | |
|---|---|---|
| Drug | 0.1 | % w/v |
| Bentone 38 (1) | 1.5 | % w/v |
| Propylene Carbonate | 0.6 | % w/v |
| Pharmasorb (2) | 10 | % w/v |
| Phosphoric Acid (3) | 0.1 | % w/v |
| Ampicillin Trihydrate | 6.0 | % w/v as free acid |
| Soya-Bean Oil | to 100 | % |

(1) Bentone 38 is an amide derivative of bentonite

(2) Pharmasorb is a brand of activated Attapulgite,

(3) The phosphoric acid is present to balance the alkaline pH of the bentone.

The Bentone is dispersed in the soya-bean oil, and when thoroughly distributed, the propylene carbonate is added with high speed mixing, followed by colloid milling to produce the base. Into this base is first mixed the phosphoric acid, and then the pharmasorb, the penicillin, and the drug and the resultant suspension is then passed through a colloid mill once more.

Claims

1. A compound of formula (I):

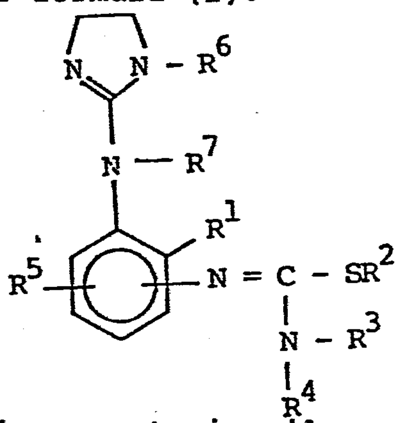

(I)

or pharmaceutically or veterinarily acceptable acid addition salts thereof,

wherein

$R^1$ is halogen, $C_{1-4}$ alkyl or $C_{1-4}$ alkoxy;
$R^2$ is hydrogen, $C_{1-4}$ alkyl or optionally substituted aryl;
$R^3$ and $R^4$ are the same or different and each is hydrogen, $C_{1-6}$ alkyl, optionally substituted aryl or optionally substituted aralkyl;
$R^5$ is hydrogen, halogen, $C_{1-4}$ alkyl or $C_{1-4}$ alkoxy and
$R^6$ and $R^7$ are the same or different and each is hydrogen, $C_{1-4}$ alkyl or acyl.

2. A compound as claimed in claim 1, wherein $R^1$ is halogen or $C_{1-4}$ alkyl.

3. A compound as claimed in claim 1 or claim 2 wherein $R^5$ is in the ortho or meta position with respect to the imidazolinamino moiety.

4. A compound as claimed in any one of claims 1 to 3, wherein $R^5$ is halogen or $C_{1-4}$ alkyl.

5. A compound as claimed in any one of claims 1 to 4, wherein the group $-N=C-SR^2$ is in the para

$$\begin{array}{l} -N=\underset{\displaystyle NR^3R^4}{\overset{|}{C}}-SR^2 \end{array}$$

position with respect to the imidazolinamino group.

6. A compound of formula (I) as defined in claim 1 selected from the group consisting of:

2-[2,6-dimethyl-4-(1-methyl-3-thioureido)phenylimino]-imidazolidine;

2-[2-methyl-5-(1-methyl-3-thioureido)phenylimino]-imidazolidine;

2-[2,6-dimethyl-4-(1-[4-flourobenzyl]-3-thioureido)-phenylimino]imidazolidine;

2-[2,6-dichloro-4-(1-methyl-3-thioureido)phenylimino]-imidazolidine;

2-[2,6-dichloro-4-(1-[4-flourobenzyl]-3-thioureido)-phenylimino]imidaxolidine;

2-[4-(1-benzyl-3-thioureido)-2,6-dimethylphenylimino]-imidazolidine;

2-[2,6-dichloro-4-(1,1-dimethyl-3-thioureido)-phenylimino]imidazolidine;

2-[2,6-dimethyl-4-(1,2-dimethyl-3-isothiouronio)-phenylimino]imidazolidine;

2-[5-(1,2-dimethyl-3-isothiouronio)-2-methyl)-phenylimino]imidazolidine;

2-[2,6-dichloro-4-(1-benzyl-3-thioureido)phenylimino] imidazolidine,

2-(2,6-dichloro-4-(1-phenyl-3-thioureido)phenylimino] imidazolidine,

2-[2,6-dichloro-4-(1-cyclohexyl-3-thioureido) phenylimino]imidazolidine,

2-[2,6-dichloro-4-(1,2-dimethyl-3-isothiouronio)-phenylimino]imidazolidine;
2-[2,6-dichloro-4-(1,1,2-trimethyl-3-isothioureido)-phenylimino]imidazolidine; and
2-[2,6-dichloro-4-(1-[4-fluorobenzyl]-2-methyl-3-isothioureido)phenylimino]imidazolidine.

7. A process for producing a compound of formula (I) or pharmaceutically or veterinarily acceptable acid addition salts thereof, as defined in claim (I), which process comprises:

(a) reacting a compound of formula (II)

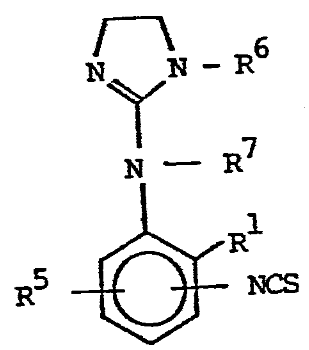

(II)

wherein $R^1$, $R^5$, $R^6$ and $R^7$ are as defined in relation to compounds of formula (I),

with an amine of formula (III):

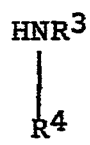

(III)

wherein $R^3$ and $R^4$ are as defined in relation to compounds of formula (I); or

(b) for compounds of formula (I) wherein $R^4$ is hydrogen, by reacting a compound of formula (IV)

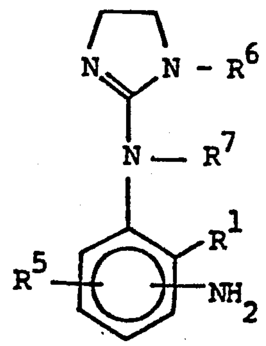

(IV)

wherein $R^1$, $R^5$, $R^6$ and $R^7$ are as defined in relation to compounds of formula (I),

with a compound of formula (V)

$$SCN-R^3 \qquad (V)$$

wherein $R^3$ is as defined in relation to compounds of formula (I); to produce a thiourea of formula (I) and optionally thereafter converting the thiourea into an isothiourea of formula (I).

8. A compound of formula (II) or acid addition salts thereof:

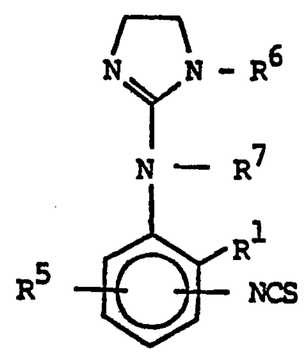

(II)

wherein $R^1$, $R^5$, $R^6$ and $R^7$ are as defined in relation to compounds of formula (I), provided that when $R^6$ and $R^7$ are hydrogen and $R^1$ and $R^5$ are chloro and $R^5$ is in the ortho position with respect to the imidazolinamino group then the isocyanato group is in the meta position with respect to the imidazolinamino group.

9. A pharmaceutical or veterinary formulation comprising a compound of formula (I) or an acid addition salt thereof, as defined in claim 1, and a pharmaceutically or veterinarily acceptable carrier therefor.

10. A compound of formula (I), as defined in claim 1, for use as an active therapeutic substance.